# EUROPEAN PATENT APPLICATION

(11) **EP 3 744 294 A1**
(43) Date of publication of application: **02.12.2020**
(21) Application number: 19176966.0
(22) Date of filing: 28.05.2019
(51) Int. Cl.: A61F 11/06

(54) **HEARING PROTECTION EQUIPMENT AND SYSTEM WITH TRAINING CONFIGURATION**

(71) Applicant: 3M Innovative Properties Company, Saint Paul, MN 55133-3427 (US)
(72) Inventor: Watson, Mr. Benjamin, Odiham, Hampshire, RG291AB (GB); Saleem, Mr. Mohammed, Reading, Berkshire, RG6 4AH (GB); Johansson, Mr. Magnus, 554 38 Jonkoping (SE)
(74) Representative: Bergen, Katja

(57) **Abstract**

The invention relates to a system comprising a hearing protection device 13 configured to be worn by a user 10; one or more audio output devices 26 configured to generate one or more audio signals, a computing device 60 comprising a memory and one or more computer processors, wherein the memory comprises instructions that when executed by the one or more computer processors cause the one or more computer processor to:
- select a training configuration 25 that defines a set of audio events 74A that correspond to a set of user reactions 74B,
- send a set of control signals to the one or more audio output device 26 that cause the one or more audio output devices 26 to simulate the set of audio events 74A,
- receive reaction data 74C that indicates whether the user 10 provided the set of user reactions 74B to the set of audio events 74A; and
- perform at least one operation based at least in part on whether the user 10 provided the set of user reactions 74B to the set of audio events 74A while wearing the hearing protection device 13.

## Description

The present disclosure relates to the field of personal protective equipment. More specifically, the present disclosure relates to a system comprising a hearing protection device and a computing device, wherein the system ensures that users wearing the hearing protection device get trained and wear the optimal hearing protection device for the environment they are working in.

Many work environments include hazards that may expose people working in to a safety event, such as a fall, breathing contaminated air, temperature related injuries (e.g. heat stroke, frostbite, etc.) and the like. Users may utilize personal protective equipment (PPE) to help protect them from harm or injury. A user may not recognize an impeding safety event until the environment becomes too dangerous or the user's health deteriorates too far.

One example of personal protective equipment or personal hearing protection device is hearing protection equipment (HPE) that is configured to be worn by a user in order to protect the user's ability to hear from a acute or long-term damage. At the same time it is important that a user wearing hearing protection equipment is still able to hear important noises around him in order to not isolate him and to make sure that the user remains connected to the world around him all the time. This is for example important to make sure, that the user is still able to hear things approaching that may bring him into a dangerous situation.

Thus, there is still a need to ensure that users wearing hearing protection are not isolated from the workplace or important communication with their peers, management or other workplace notifications.

The present invention provides a system comprising
- a hearing protection device configured to be worn by a user,
- one or more audio output devices configured to generate one or more audio signals,
- a computing device comprising a memory and one or more computer processors,
wherein the memory of the computing device comprises instructions that when executed by the one or more computer processors cause the one or more computer processor to:
- select a training configuration that defines a set of audio events that correspond to a set of user reactions,
- send a set of control signals to the one or more audio output devices that cause the one or more audio output devices to simulate the set of audio events,
- receive reaction data that indicates whether the user provided the set of user reactions to the set of audio events; and
- perform at least one operation based at least in part on whether the user provided the set of user reactions to the set of audio events while wearing the hearing protection device.

In other words, the present invention provides a system with a hearing protection device, one or more audio output devices and a computer device. The system is configured such that when a user is wearing the hearing protection device the system can be used as a training system for the user wearing the hearing protection device. The training system may be used for a variety of different trainings, such as for example but not limited for
a) training a user to get used to a new hearing protection device in a known working environment;
b) training a user to get used to wearing a known hearing protection device in a new working environment;
c)helping a user to identify the optimal hearing protection device for a certain environment and so on; or
d) helping a user to identify the origin of an acoustic event.

The hearing protection device may be any kind of hearing protection device, that is configured to be worn by a user and to cover at least one ear. The hearing protection device may also be worn in at least one ear. Hearing protection devices reduce (not eliminate the level of the noise entering the ear). There are many different types of hearing protection devices available for use, including earmuffs, earplugs, electronic hearing protection devices and semi-insert devices. The audio output device may be any kind of device that is able to generate one or more audio signals, such as for example loud speakers or the like. The audio output devices will be described in more detail below. Those audio signals may be any kind of audio signals, especially audio signals that resemble to noises or acoustic events occurring during a working environment of a user. With these audio signals it is possible to simulate a working environment of a user.

The computing device comprising a memory and one or more processors may be any kind of computing device providing these components. It may be a stand alone computing device with memory and processor or it may be a computing device that is integrated into a combination of the hearing protection equipment and in a wider a communications infrastructure, wherein the communications infrastructure may be a network of several computing devices that are connected with each other through all known communication connections between computing devices such as connection through wires or wireless connections.

The memory of the computing device of the training system according to the invention comprises instructions that when executed by one or more computer processors cause the processors to run through the following steps: first a training configuration is selected. The training configuration may be a safety training configuration. The training defines a set of audio events, which may simulate audio events as they occur in different environments, for example working environments. The training configuration also provides a set of possible user reactions related to each of those audio events. As a second step the computer processor sends out a set of control signals to the one or more audio output devices to cause the one or more audio output devices to simulate or generate the set of audio events corresponding to the selected training configuration. As a next step the computer processor may receive a reaction data that indicates if a user, wearing the hearing protection device and using the system for a training provided a reaction that corresponds to the set of user reactions being part of the selected training configuration. The reaction data may be generated through gesture recognition or other input devices as will be described in more detail below. The user reactions will be described in more detail further below. And finally, the computer processor performs at least one operation based at least in part on whether the user provided the set of user reactions to the set of audio events of the selected training configuration while wearing the hearing protection device. The at least one operation may be one selected from a variety of possible operations and will also be described in more detail in the following.

Thus, the training system according to the invention simulates audio experiences or audio events that simulate real live audio experiences of a environment and helps to get users used to hear these acoustic experiences through hearing protection devices. The system may be used to train a user to get used to a new hearing protection device, to get used to a new working environment and also to train according reactions to a certain acoustic experience.

As already mentioned above, there is still a need to ensure users wearing hearing protection devices are not isolated from the workplace or important communication with their peers, management or other workplace notification. The training system according to the invention provides a basis to not only train a user wearing hearing protection devices but also monitor levels of fatigue by measuring response times from within the training application. Pre-training users wearing hearing protection will enable them to more accurately detect safety critical changes in contrast to hearing those changes for the first time during operational use. Pre-training wearers of hearing protection and related personal protection equipment will enable them to more quickly enter their environment with prior knowledge of how their work environment might sound through their hearing protection devices, where the sound originates from and how to respond more quickly. And finally, the system leverages digital technologies with hearing protection devices during a training process, to improve performance with increased safety benefits that avoid isolation, whilst helping the wearer to identify the location of important audible changes and messages within their environment.

According to one embodiment of the training system according to the invention the memory of the computing device comprises instructions that when executed by the one or more computer processors causes the one or more computer processors to send out a set of control signals to the one or more audio output device that cause the one or more audio output devices to simulate the set of audio events, wherein the control signals are configured to cause the audio output device to generate at least one audio output signal that provides an audio experience, for example a two-dimensional and/or a three-dimensional experience, to a user. When the audio output signal provides a three-dimensional audio experience is generated a much more realistic simulation of an environment is possible which helps to provide a much more effective training for a user.

The memory of the computing device of the training system may also comprise instructions that when executed by the one or more computer processors causes the one or more computer processors to select a safety training configuration that defines a set of audio events that correspond to a set of user reactions, send out a set of control signals to the one or more audio output devices that cause the one or more audio output devices to simulate a set of audio events at one or more particular locations in a three-dimensional space around the user, receive reaction data that indicates whether the user provided the set of user reactions to the set of audio events at the one or more particular locations and perform at least one operation based at least in part on whether the user provided the set of user reactions to the set of audio events at the one or more particular locations while wearing the hearing protection device. The audio events may for example be delivered to the user in a combination of different sequences, for example in a different running order for the sound/audio event. The training system may for example be installed in a training room, wherein the audio output devices are positioned for example as loud speakers in one or more locations in this training room to simulate the three-dimensional audio experience around the user. A user who uses the system needs to go into this training room. As soon as the training configuration is started, the system will send out control signals to the audio output devices in the training room that needs to be identified by the user (user reactions). The training room may be equipped with additional features for example devices for receiving the reaction data, as will be described in more detail in the following.

As an alternative, the audio output devices that creat the set of audio events may be integrated into the hearing protection device to simulate the three-dimensional audio experience. For such a configuration the audio output devices may be little loud speakers that may be integrated into the hearing protection device. With such a system, it is not necessary to set up a training room with audio output devices. The training system may be used anywhere.

According to another embodiment of the training system according to the invention, the memory of the computing device may comprise instructions that when executed by the one or more computer processors cause the one or more computer processor to select a safety training configuration that defines a set of audio events that correspond to a set of user reactions, to send a set of control signals to the one or more audio output devices that cause the one or more audio output devices to simulate the set of audio events, wherein the set of audio events simulate real-life three-dimensional audio experience that may occur during the day work of a user. The users may for example be wood workers, workers working in the heavy metal industry or street workers. Depending on the selected training configuration the system may generate the according audio experience (noises that occur during a working day of a wood worker, noises that occur during a working day of a heavy metal worker, noises that occur during a working day of a street worker).

According to another embodiment of the training system according to the invention, the memory of the computing device may comprise instructions that when executed by the one or more computer processor causes the one or more computer processor to reference the set of audio events to a set of user reactions identifying the according location in a three-dimensional space around the user where the audio experience comes from. Thus, the system may expose a user to a set of audio events and the user needs to identify the location where the noise comes from. With such a training system the user may get trained to localize a noise. It is also possible that the set of audio events gets referenced to a set of user reaction identifying the kind of audio event. The system may be configured such that a user needs to identify both the location and the kind of noise. The system may also be configured such that the user identifies either the location or the kind of noise.

For identifying the audio event, the user may for example need to detect the origin of the audio event. The user may for example point towards the location of origin or use other gestures for identification. According to the second aspect, it is also possible that the user needs to identify the kind of noise, for example by using a certain gesture for certain kind of noises.

The system may also be configured such that the memory of the computing device comprises instructions that when executed by the one or more computer processors cause the one or more computer processors to reference that set of audio events to a set of defined time frames for the time passing between sending out the set of control signals to the one or more audio output devices that cause the one ore more audio output devices to simulate the set of audio events and receiving reaction data that indicate whether the user provided the set of user reactions. With these time frames a user can be trained to provide a certain reaction within a certain time frame. This may be helpful in situations where the noise indicates for example an approaching object and a user would have to move away. Thus, the user may get trained on a timely reaction towards an acoustic event. With such time frames a "gesture window" may be defined, which may become a basis for measuring the time and the accuracy of a user reaction. The gesture window may for example be used to compare data of one user with other data, for example with data of peer users.

Summarizing the above, the data point of the reaction data may comprise data about the time that has passed between sending out the set of control signals to the one or more audio output devices that causes the one or more audio output devices to simulate the set of audio events and receiving reaction data that indicates whether the user provided the set of user reactions as well as data about the accuracy of detection, which may include accuracy of determining the location and accuracy of determining the kind of audio event.

The training system may also provide instructions that when executed by the one or more computer processors causes the one or more computer processors to receive reaction data that indicate whether the user provided the set of user reactions to the set of audio events at the one or more particular locations and use the reaction data to create a profile of at least one user. The profile of the user may be used for several purposes such as for example comparison of profiles of several users or comparison of one profile of a user to see if the profile changes over time.

The at least one operation that is performed by the one or more computer processors of the training system as a last step and that is based at least in part on whether the user provided the set of user reactions to the set of audio events while wearing the hearing protection device may for example be selected from one or more of the following operations:
- providing a predefined feedback to the user,
- providing a selection of an appropriate next safety training configuration,
- providing a suggestion of an appropriate hearing protection device,
- creating a profile of a user with a number of reaction data,
- providing information packages for a safety management of the user,
- providing a comparison of reaction data of one user with reaction data of another user,
- providing a comparison of reaction data of one user with the profile of this user.

The operation of providing a predefined feedback to the user may be realised for example through an audio information to the user through the one or more audio output devices. It may also be realised through any other kind of information transmitting device such as a display or an optical device such as a light generating device. All these devices may be communicatively connected to the computing device and may receive according signals from the one or more processors.

The operation of providing a selection of appropriate next training configurations for example safety training configurations may be realised through an audio information or through an optical information. The computing system may provide this information through its one or more computer processors and send it to any known information output device that may be connected to the computer device. The next safety training configuration may for example be a next level of training configuration including further training aspects. The information send to the user may also include the timeframe within which the user should conduct the next training configuration and also the frequency.

The at least one operation may also include providing a suggestion of an appropriate hearing protection device. Depending on the received reaction data, it may as well be, that a more suitable hearing protection device exists for a certain working environment and a specific user. The system may therefore provide a suggestion for another more suitable hearing protection device. The system may also provide a suggestion for another hearing protection device in combination with another safety training configuration to also test the proposed hearing protection device for the specific user.

Another possibility for an operation that is performed by the system may be the creation of a profile of a user with a number of reaction data. This profile may for example be used in a safety management system. It may be used for example to document that a user is protected with the correct hearing protection device. It may also be used to compare date of one user with peer users. It may also be used to review the profile of a user over time in order to be able to detect any deviation that may come up over time. This is a possibility to be able to identify non-typical changes in reaction data, that may for example be an indication of a compliance issue or of a fatigue situation.

All the above-mentioned operations are examples only and it is possible that the system performs any other kind of operation. It is also possible that not only one of the operations will be performed but a combination of operations.

It is also possible that the system according to the invention comprises instructions that when executed by the one or more computer processors cause the one or more computer processors to select a safety training configuration that defines a set of audio events that correspond to a set of user reactions, wherein the set of audio events is directed to an off-site training which is configured such as to train users to get used to a hearing protection device that they have not used before. Off-site training may be a training that is conducted off-site of a working environment simulating audio signals of a real-life working environment in order to train a user to a new hearing protection device. It is also possible that the set of audio events is directed to an on-site training which is configured such as to train a user to get used to an environment that he has not worked in before. On-site training may be a training that is conducted in a real working environment and that helps a user to get used to a new working environment. And finally may the set of audio events be directed to an on-site on the job training which is configured such as to check if the user is wearing the appropriate hearing protection device.

As already mentioned above, the hearing protection device may be any kind of known hearing protection device worn in or over at least one ear while exposed to hazardous noise to help prevent noise-induced hearing loss. The hearing protection device may be an active hearing protection device including electronic features or it may be a passive inactive hearing protection device. It may be part of a protection system including other personal protection devices, such as for example respirators or eye protection devices.

The hearing protection device may be communicatively coupled to the computing device. This may be beneficial because the hearing protection device may communicatively be integrated into the training system. Depending on the hearing protection device it may for example be used for integrating audio output devices and generating audio signals. It may also be used to perform at least one operation such as for example providing feedback to the user or proposing another hearing protection device or another training configuration. Finally, it may be used to integrate any devices that might be necessary to receive reaction data as will be described in more detail further down.

It is also possible that the hearing protection device is equipped with communication devices such as for example a head set. A hearing protection device with a head set may be used for generating audio signals generated by the safety training configuration, for performing at least one operation and also for receiving audio information from the user.

As already mentioned above it may be possible to integrate audio output devices in the hearing protection equipment, which would make it possible to use them everywhere and not only in a defined training room. The integration of audio output devices that are able to generate a two-dimensional and/or a three-dimensional audio experience may be achieved for example through an array of miniature speakers built into the hearing protection device. It is also possible to apply signal processing to create the two-dimensional and/or three-dimensional experience. The audio output devices may also be positioned in a three-dimensional training space or room. They may for example be provided through a known three-dimensional audio system arranged in a training space.

The system may also comprise a reaction recognition device which is communicatively coupled to the computing device and which provides input signals for the received reaction data. The reaction recognition device may be a separate device or it may be integrated into components of the training system such as for example the hearing protection device. The reaction recognition device needs to be configured such that it is able to recognize a reaction of a user. It may recognize the reaction of a user for example through recognizing a predefined gesture or movement of a user. It should also be able to log the time at which it recognizes the reaction of the user. One example of a reaction recognition device may be a camera. It may also be an acceleration sensor. The reaction recognition device may be configured such that it can be held in a user's hand or that it can be fixed to a user's equipment or body.

The invention will now be described in more detail with reference to the following Figures exemplifying particular embodiments of the invention:
- Fig. 1: is a block diagram illustrating an example of a safety training system, in accordance with various techniques of this disclosure;
- Fig. 2: is a block diagram illustrating, in detail, an operating perspective of the system that includes the safety training configuration shown in figure 1; and
- Fig. 3: is a flow-chart illustrating an example of a safety training configuration of the system shown in figure 1.

Herein below various embodiments of the present invention are described and shown in the drawings wherein like elements are provided with the same reference numbers.

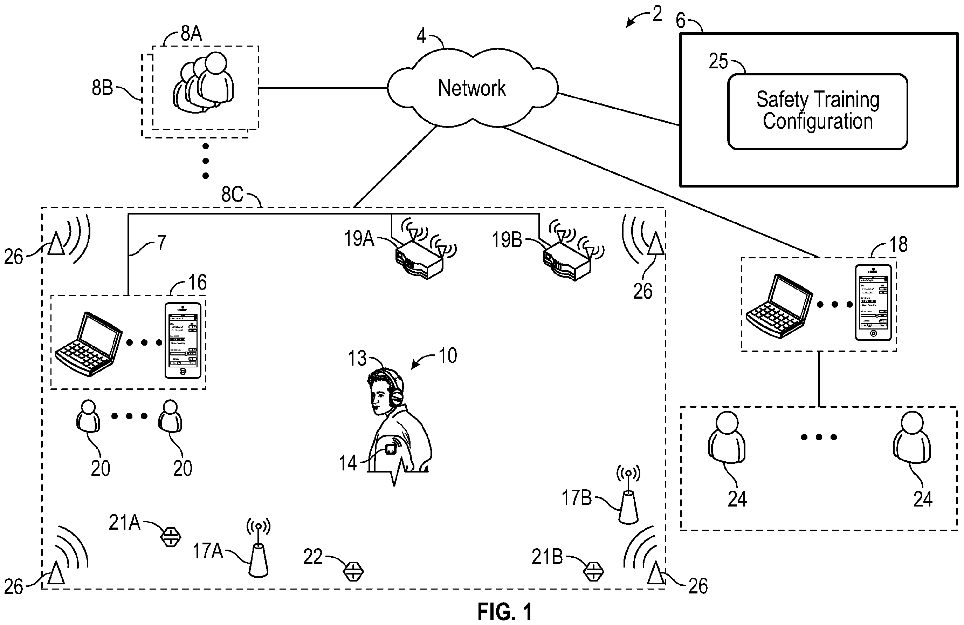

Fig. 1 is a block diagram illustrating an example system 2 that includes a safety training system 6 for training or educating a user that needs to wear a hearing protection equipment 13. In general, the safety training system 6 provide data acquisition, monitoring, activity logging, reporting, predictive analytics, safety condition identification and alert generation. Especially the safety training system 6 comprises a safety training configuration 25 with a set of audio events that correspond to a set of user reactions as will be described in more detail below.

As shown in figure 1, system 2 represents a computing environment in which computing device(s) with a plurality of physical environments 8A, 8B, 8C (collectively, environments 8) electronically communicate with the safety training system 6 via one or more computer networks 4. Each physical environment 8 represents a physical environment, such as a work or a training environment, in which one or more individuals, such as users 10, utilize personal protective equipment 13 while engaging in tasks or activities with the respective environment.

In this example, environment 8A and 8B are shown generally as having users 10, while environment 8C is shown in expanded form to provide more detailed example and has only one user 10.

As shown in the example of figure 1, an environment, such as environment 8C, may also contain one or more wireless-enabled beacons, such as beacons 17A, 17B, that provide accurate location data within the work environment. For example, beacons 17A, 17B may be a GPS-enabled such that a controller within the respective beacon may be able to precisely determine the position of the respective beacon. Based on wireless communications with one or more of beacons 17, a given hearing protection device 13 or communication hub 14 worn by a user 10 is configured to determine the location of the user 10 within environment 8C. In this way, any data reported to the safety training system 6 may be stamped with positional data to aid analysis, reporting and analytics performed by the safety training system 6.

In addition, an environment, such as the environment 8C, may also include one or more wireless-enabled sensing stations, such as sensing stations 21A, 21B, 21C. Each sensing station 21 includes one or more sensors and a controller configured to output data indicative of sensed environmental conditions. Moreover, sensing stations 21 may be positioned within respective geographic regions of environment 8 or otherwise interact with beacons 17 to determine respective positions and include such positional data when reporting environmental data to safety training system 6. Example environmental conditions that may be sensed by sensing stations 21 include but are not limited to temperature, humidity, presence of gas, pressure, visibility, wind and the like.

In the example of figure 1, the user 10 is shown as utilizing a hearing protection device 13. A hearing protection device is an ear protection device worn in or over the ears while exposed to hazardous noise to help prevent noise-inducted hearing loss. Hearing protection devices reduce (not eliminate) the level of the noise entering the ear. There are many different types of hearing protection devices available for use, including earmuffs, earplugs, electronic hearing protection devices and semi-insert devices. The user may also wear other personal protection devices such as respirators, vision protection devices, head protection devices and so on.

The hearing protection device 13 as well as the other mentioned personal protection devices may include embedded sensors or monitoring devices and processing electronics configured to capture data in real-time as a user (e.g. a user) engages in activities while utilizing (e.g. wearing) the hearing protection device 13 or others. The hearing protection device 13 may include a number of equipment sensors for sensing or controlling the operation of such components.

In addition, each hearing protection device 13 may include one or more output devices for outputting data that is indicative of the operation of the hearing protection device 13 and/or generating and outputting communications to the respective user 10. For example, hearing protection device 13 may include one or more devices to generate audible feedback (e.g. one or more speakers), visual feedback (e.g., one or more displays, light emitting diodes (LEDs) or the like), or tactile feedback (e.g. a device that vibrates or provides other haptic feedback).

In general, each of environments 8 include computing facilities (e.g., a local area network) by which physiological sensors 22, sensing stations 21 beacons 17, and/or hearing protection device 13 are able to communicate with the safety training system 6. For example, environments 8 may be configured with wireless technology. In the example, environment 8C includes a local network 7 that provides a packet-based transport medium for communicating with the safety training system 6 via network 4. Environment 8 may include a wireless access point 19 to provide support for wireless communications. In some examples and depending on the size of the environment, environment 8 may include a plurality of wireless access points 19 that may be distributed throughout the environment 8C to provide support for wireless communications throughout the environment.

In some examples, the user 10 may be equipped with respective one of wearable communication hubs 14 that enable and facilitate wireless communication. And each of environments 8 may include computing facilities that provide an operating environment for end-user computing devices 16 for interacting with the safety training system 6 via network 4. Similarly, remote users may use computing devices 18 to interact with safety training system 6 via network 4. For purpose of example, the end-user computing device 16 may be laptops, desktop computers, mobile devices such as tablets or smart phones and the like.

In one example, environment 8 may provide one or more audio output devices 26. The audio output devices may be communicatively coupled to the safety training system 6 via the network 4. The one or more audio output devices may be selected and configured such that they are able to create a three-dimensional audio experience to the user. The safety training system 6 may send out control signals to the one or more audio output devices 26 in order to cause the one or more audio output devices to simulate a set of audio events. The audio output devices 26 may also be integrated into the hearing protection device 13 such that they are able to create a three-dimensional acoustic experience to the user (not shown in the drawings).

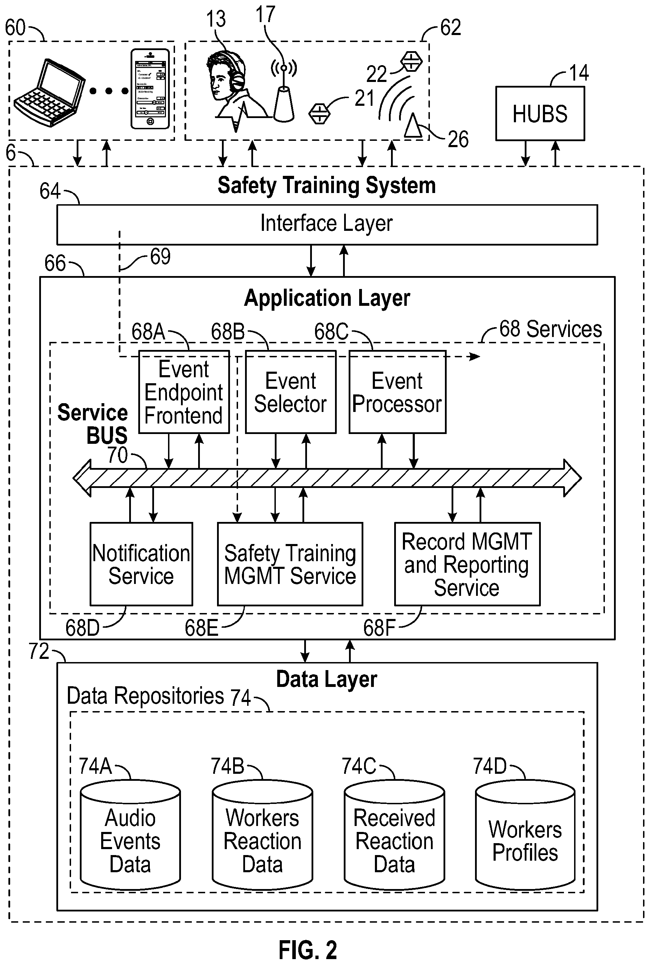

Figure 2 is a block diagram providing an operating perspective of the system according to the invention, especially of the safety training system 6 with the safety training configuration 25, when hosted as cloud-based platform capable of supporting multiple, distinct environments 8. In the example of figure 2, the components of the safety training system 6 are arranged according to multiple logical layers that implement the techniques of the disclosure. Each layer may be implemented by one or more modules comprised of hardware, software, or a combination of hardware or software.

In figure 2, safety equipment 62 includes personal hearing protection devices 13, beacons 17, sensing stations 21, physiological sensors 22 and audio output devices 26. Safety equipment 62, HUBs 14, as well as computing devices 60 operate as clients 63 that communicate with the safety training system 6 via an interface layer 64. Computing devices 60 typically execute client software applications, such as desktop applications, mobile applications, and web applications. Computing devices 60 may represent any of computing devices 16, 18 of figure 1. Examples of computing devices may include but are not limited to a portable or mobile computing device (e.g. smartphone, wearable computing device, or tablet), laptop computers, desktop computers, smart television platforms, and servers, to name only a few examples.

Client applications executing on computing devices 60 may communicate with the safety training system 6 to send and receive data that is retrieved, stored, generated, and/or otherwise processed by services 68. For instance, the client applications may request and edit safety training configurations 25 including audio events and users reactions stored and/or managed by the safety training system 6. In some examples, client applications may request and display of audio events and according users reactions. The client applications may interact with safety training system 6 to query for analytics data about past and predicted safety training configurations and received reaction data. In some examples, the client applications may output for display data received from safety training system 6, such as reaction data. As further illustrated and described in below, safety training system 6 may provide data to the client application, which the client applications output for display in user interfaces.

Client applications executing on computing devices 60 may be implemented for different platforms but include similar or the same functionality. For instance, a client application may be a desktop application compiled to run on a desktop operating system or a mobile application compiled to run on a mobile operating system.

As shown in figure 2, safety training system 6 further includes an interface layer 64 that is represented and supported by the safety training system 6. Interface layer 64 initially receives messages from any of clients 63 for further processing at the safety training system 6. Interface layer 64 may therefore provide one or more interfaces that are available to client applications executing on clients 63. In some examples, the interfaces may be application programming interfaces (APIs) that are accessible over a network. Interface layer 64 may be implemented with one or more web servers. The one or more web servers may receive incoming requests, process and/or forward data from the requests to services 68 (services will be described in more detail further below) inside of the safety training system 6 and provide one or more responses based on the data received from services 68, to the client application that initially sent the request. In some examples, the one or more web servers that implement interface layer 64 may include a runtime environment to deploy program logic that provides the one or more interfaces. As further described below, each service may provide a group of one or more interfaces that are accessible via interface layer 64.

As shown in figure 2, safety trainings system 6 also includes an application layer 66 that represents a collection of services for implementing much of the underlying operations of the safety training system 6. Application layer 6 receives data included in requests received from client applications and further processes the data according to one or more of services 68 invoked by the requests. Application layer 66 may be implemented as one or more discrete software services executing on one or more application servers, e.g. physical or virtual machines. In some, examples, the functionality interface layer 64 as described above and the functionality of application layer 66 may be implemented at the same server.

Application layer 66 may include one or more separate software services 68, e.g. processes that communicate, e.g. via a logical service bus 70 as one example. Service bus 70 generally represents logical interconnections or set of interfaces that allows different services to send messages to other services, such as by a publish/subscription communication model. For instance, each of services 68 may subscribe to specific types of messages based on criteria set for the respective service. When a service publishes a message of a particular type on service bus 70, other services that subscribe to messages of that type will receive the message. In this way, each of services 68 may communicate data to one another. As another example, services 68 may communicate in point-to-point fashion using sockets or other communication mechanisms. Before describing the functionality of each service 68, the layers are briefly described herein.

Data layer 72 of the safety training system 6 represents a data repository that provides persistence for data in the safety training system 6 using one or more data repositories 74. Data repository, generally, may be any data structure or software that stores and/or manages data. Examples of data repositories include but are not limited to relational databases, multi-dimensional databases, maps, and hash tables, to name only a few examples. In the safety training system 6 according to the invention the data repositories may for example provide audio event data 74A, set of user reaction data 74B, received reaction data 74C or users profiles 74D.

As shown in figure 2, each of the services 68A to 68F is implemented in a modular form within the safety training system 6. Although shown as separate modules for each service, in some examples the functionality of two or more services may be combined into a single module or component. Each of services 68 may be implemented in software, hardware or a combination of hardware and software. Moreover, services 68 may be implemented as standalone devices, separate virtual machines or containers, processes, threads or software instructions generally for execution on one or more physical processors. In some examples, one or more of services 68 may each provide one or more interfaces that are exposed through interface layer 64. Accordingly, client applications of computing devices 60 may call one or more interfaces of one or more of services 68 to perform techniques of this disclosure.

In accordance with techniques of the disclosure, services 68 may include an event endpoint frontend 68A, event selector 68B, an event processor 68C, a notification service 68D, a safety training management service 68E and/or a record management and reporting service 68F.

Event endpoint frontend 68A operates as a frontend interface for exchanging communications with hubs 14 and safety equipment 62. In other words, event endpoint frontend 68A operates to as a frontline interface to safety equipment deployed within environments 8 and utilized by users 10. Each incoming communication may, for example, carry data recently captured representing sensed conditions, motions, temperatures, actions or other data, generally referred to as events. Communications exchanged between the event endpoint frontend 68A and safety equipment 62 and/or hubs 14 may be real-time or pseudo real-time depending on communication delays and continuity.

Event selector 68B operates on the stream of events 69 received from safety equipment 62 and/or hubs 14 via frontend 68A and determines, based on rules or classifications, priorities associated with the incoming events. Based on the priorities, event selector 68B enqueues the events for subsequent processing by event processor 68C. In general, event processor 68C operate on the incoming streams 69 to update data within the data repository 74. For example, users reaction data 74C or users profiles 74D may be updated. User reactions 74B may include information identifying the according location of an audio event in a three-dimensional space and information identifying the kind of acoustic experience of the audio event. In other instances, the users reaction 74B may include information about the time that has passed between sending out a set of audio events and receiving reaction data.

The event processor 68C may create, read, update, and delete received reaction data 74C or users profiles 74D. Received reaction data 74C may include name/value pairs of data, such as data tables specified in row/column format.

In accordance with the techniques of this disclosure, safety training management service 68Emay determine a safety training configuration for one or more respective users 10 based at least in part on events within event stream 69. Safety training management service 68E may be configured to determine the according safety training configuration for a user 10 based at least in part on a selection of the user 10 itself or the safety management of the user 10, selected data of a user 10 and one or more rules. Although other technologies can be used, in examples, the one or more rules are generated using machine learning. In other words, in one example implementation, safety training management service 68Eutilizes machine learning when operating on event streams 69 so as to perform real-time analytics. That is, safety training management service 68E may include executable code generated by application of machine learning to determine a safety risk score for the user.

In the following one example of an application of the safety training system 6 according to the invention will be described referring to the figures 1 and 2. A user 10 that may be exposed to a new working environment 8 and a new hearing protection device 13 will be exposed to some training before entering the real-life working environment. For this purpose, the user 10 may be equipped with a suitable hearing protection device 13. The user will put on the suitable hearing protection device 13 and he will get exposed to audio events. The audio events may be selected such that they simulate audio events that occur in a real live working environment of the user.

There are different ways how the user might get exposed the audio events. One example is that the user needs to enter a special training room or space 8 together with the hearing protection device 13. This training room or space 8 may for example be equipped with audio output devices 26 in the form of loud speakers. The audio output devices 26 may further be communicatively connected with the training system 6 according to the invention and may be used to generate the audio events for the user 10.

Another possibility is that the audio output devices are little loud speakers that are integrated into the hearing protection device 13. Such a configuration provides the advantage that no special training room 8 is required and that the training can be done anywhere.

As soon as the user 10 has entered the training room 8 and put on the hearing protection device 13 - or for the second example as soon as the user 10 has put on the hearing protection device 13 - and as soon as someone has started the training system, a training configuration is selected. The training configuration may include a series of audio events 74A that resemble for example audio events 74A of real-live working environments. The training configurations may exist in different levels, such as for example a starter level as well as several levels for more experienced users 10. The training configuration may also exist for different working environments. For each audio event 74A the training configuration may provide a set of users reactions 74B that the system expects and considers as being the right reaction of a user. Which kind of information the user reactions may include will be described in the following.

As a next step the training system 6 may send out a set of control signals to the one or more audio output devices (either separate audio output devices 26 in a training room 8 or audio output devices integrated into the hearing protection device 13), that cause the one or more audio devices to simulate a set of audio events 74A. According to the training rules the user 10 needs to concentrate on those audio events 74A and identify them. Thus, the user 10 needs to react in a certain (see set of user reaction 74B above) way to each of the audio events 74A.

The training system 6 will as a next step receive a reaction data 74C in a certain way, wherein the reaction data 74C indicates whether the user provided the set of user reactions 74B to the set of audio events 74A mentioned before or if he did not provide the set of user's reactions.

Depending on the received reaction data 74C the training system 6 will perform at least one operation based at least in part on whether the user provided the set of user reactions to the set of audio events while wearing the hearing protection device 13. Depending on the reason for the training, the operations may be very different. For the above described case, where a user is trained on a new working environment and a new hearing protection device, the selected operations may for example be to first save the received reaction data in the data layer 72 of the system 6. The training system 6 may further provide direct feedback to the user 10 for example by indicating if the user 10 came up with a user reactions 74C that resembles to the set of user reactions 74B that the training configuration includes. Or the system 6 may provide a feedback if the users' reactions 74C do not resemble to the set of user reactions 74B.

Another possible operation of the training system 6 may be the proposal of an appropriate next level safety training configuration that considers the results of the training just absolved.

The training system 6 may also propose another hearing protection device, if for example the received reaction data 74C provides information that the user is not well enough connected to the world outside of the hearing protection devices.

The system may also create a profile 74D of the user and store it in the data layer 72. A profile of a user 10 may be used for different purposes. One idea is to compare a profile of one user 10 with the profile of other peer users 10. Another idea is to compare a profile of a user 10 over a longer period of time to be able to detect any development. If the training is done on a frequent basis, the development may for example be used as an indicator of fatigue.

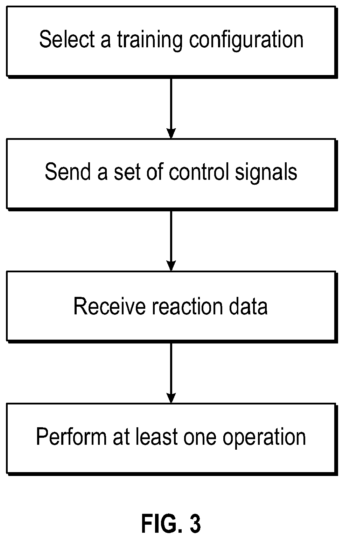

The different steps that the safety training 6 performs and that have been described above may also be seen in figure 3, where it starts with the first step of selecting a training configuration that defines a set of audio events that correspond to a set of user reactions, followed by the step of sending out control signals to one or more audio output devices that cause the one or more audio output device to simulate the set of audio events. The system may further receive reaction data from the user going through a training that indicate whether the user provided the set of user reactions to the set of audio events. And finally, the system performs at least one operation bae at least in part on whether the user provided the set of user reactions to the set of audio events while wearing the hearing protection device.

The system according to the invention may also comprise a reaction recognition device. The reaction recognition device may be a separate device or it may be integrated into components of the training system such as for example the hearing protection device. The reaction recognition device needs to be configured such that it is able to recognize a reaction of a user. It may recognize the reaction of a user for example through recognizing a predefined gesture or movement of a user. It should also be able to log the time at which it recognizes the reaction of the user. One example of a reaction recognition device may be a camera. It may also be an acceleration sensor. The reaction recognition device may be configured such that it can be hold in a user's hand or that it can be fixed to a user's equipment or body or somewhere in the space around the user.

## Claims

1. A system comprising:
- a hearing protection device 13 configured to be worn by a user 10,
- one or more audio output devices 26 configured to generate one or more audio signals,
- a computing device 60 comprising a memory and one or more computer processors, wherein the memory comprises instructions that when executed by the one or more computer processors cause the one or more computer processor to:
- select a training configuration 25 that defines a set of audio events 74A that correspond to a set of user reactions 74B,
- send a set of control signals to the one or more audio output device 26 that cause the one or more audio output devices 26 to simulate the set of audio events 74A,
- receive reaction data 74C that indicates whether the user provided the set of user reactions 74B to the set of audio events 74C; and
- perform at least one operation based at least in part on whether the user 10 provided the set of user reactions 74B to the set of audio events 74A while wearing the hearing protection device 13.

2. The system according to claim 1, wherein the memory of the computing device 60 comprises instructions that when executed by the one or more computer processors cause the one or more computer processor to:
- send a set of control signals to the one or more audio output device 26 that cause the one or more audio output devices 26 to simulate the set of audio events 74A,
- wherein the control signals are configured to cause the audio output device 26 to generate at least one audio output signal that provides a three-dimensional acoustic experience to a user 10.

3. The system according to claim 1 or claim 2, wherein the memory of the computing device 60 comprises instructions that when executed by the one or more computer processors cause the one or more computer processor to:
- select a training configuration 25 that defines a set of audio events 74A that correspond to a set of user reactions 74B,
- send a set of control signals to the one or more audio output device 26 that cause the one or more audio output devices 26 to simulate the set of audio events 74A at one or more particular locations in a three-dimensional space around the user 10 or in the hearing protection device 13 to simulate a the three-dimensional acoustic experience to the user 10,
- receive reaction data 74C that indicates whether the user 10 provided the set of user reactions 74B to the set of audio events 74A at the one or more particular locations; and
- perform at least one operation based at least in part on whether the user 10 provided the set of user reactions 74B to the set of audio events 74A at the one or more particular locations while wearing the hearing protection device 13.

4. The system according to any of the preceding claims, wherein the memory of the computing device 60 comprises instructions that when executed by the one or more computer processors cause the one or more computer processor to:
- select a training configuration 25 that defines a set of audio events 74A that correspond to a set of user reactions 74B
- send a set of control signals to the one or more audio output device 26 that cause the one or more audio output devices 26 to simulate the set of audio events 74A,
- wherein the a set of audio events 74A simulate real-life three-dimensional acoustic experience that may occur during the day of a user 10.

5. The system according to any of the preceding claims, wherein the memory of the computing device 60 comprises instructions that when executed by the one or more computer processors causes the one or more computer processors to:
- reference that set of audio events 74A to a set of user reactions 74B identifying the according location in the three-dimensional space around the user 10 where the acoustic experience comes from and/or
- reference that set of audio events 74A to a set of user reactions 74B identifying the kind of acoustic experience.

6. The system according to any of the preceding claims, wherein the memory of the computing device 60 comprises instructions that when executed by the one or more computer processors causes the one or more computer processors to:
- reference that set of audio events 74A to a set of defined time frames for the time passing between sending out the set of control signals to the one or more audio output device 26 that causes the one or more audio output device 26 to simulate the set of audio events 74A and receiving reaction data 74C that indicates whether the user 10 provided the set of user reactions 74B.

7. The system according to any of the preceding claims, wherein the at least one operation based at least in part on whether the user 10 provided the set of user reactions 74B to the set of audio events 74A while wearing the hearing protection device 13 is selected from one or more of the following operations:
- providing a predefined feedback to the user,
- providing a selection of an appropriate next training configuration,
- providing a suggestion of an appropriate hearing protection device,
- creating a profile of a user with a number of reaction data,
- providing information packages for a management of the user,
- providing a comparison of reaction data of one user with reaction data of another user,
- providing a comparison of reaction data of one user with the profile of this user.

8. The system according to any of the preceding claims, wherein the hearing protection device 13 may be any kind of known hearing protection device worn in or over at least one ear while exposed to hazardous noise to help prevent noise-induced hearing loss.

9. The system according to any of the preceding claims, wherein the hearing protection device 13 is communicatively coupled to the computing device.

10. The system according to any of the preceding claims, wherein the hearing protection device 13 may be equipped with communication devices such as for example a head set.

11. The system according to any of the preceding claims, wherein the audio output device 26 is integrated in the hearing protection equipment.

12. The system according to any of the claims 1 to 10, wherein the audio output device 26 is positioned in a training space.

13. The system according to any of the preceding claims, wherein the system also comprises a reaction recognition device, which is communicatively coupled to the computing device 60 and which is providing input signals for the received reaction data 74C.

14. The system according to any of the preceding claim, wherein the reaction recognition device is a separate device or is integrated into components of the system such as for example the hearing protection device.

15. The system according to any of the preceding claims, wherein the reaction recognition device may be configured such that it can be hold in a user's hand or that it can be fixed to a user's equipment or body.

16. The system according to any of the preceding claim, wherein the reaction recognition device, comprises a camera or an acceleration sensor.
